(19) Europäisches Patentamt
European Patent Office
Office européen des brevets

(11) Publication number: **0 359 399 B1**

(12) # EUROPEAN PATENT SPECIFICATION

(45) Date of publication of patent specification: **16.03.94**

(51) Int. Cl.$^5$: **C12P 21/02**, C07K 1/00

(21) Application number: **89308232.1**

(22) Date of filing: **14.08.89**

(54) **Process for the enzymatic production of dipeptides.**

(30) Priority: **12.08.88 DK 4521/88**

(43) Date of publication of application:
**21.03.90 Bulletin 90/12**

(45) Publication of the grant of the patent:
**16.03.94 Bulletin 94/11**

(84) Designated Contracting States:
**AT BE CH DE ES FR GB GR IT LI LU NL SE**

(56) References cited:
**EP-A- 0 017 485**
**EP-A- 0 074 095**
**EP-A- 0 154 472**
**EP-A- 0 220 923**
**EP-A- 0 269 390**
**EP-A- 0 278 787**

(73) Proprietor: **CARLBIOTECH LTD. A/S**
**Tagensvej 16**
**DK-2200 Copenhagen N(DK)**

(72) Inventor: **Aasmul-Olsen, Stig**
**Skodsborgvej 410, st.tv.**
**DK-2942 Skodsborg(DK)**
Inventor: **Thorbek, Pia**
**Sdr. Jagtvej 39**
**DK-2970 Horsholm(DK)**
Inventor: **Widmer, Fred**
**c/o Carlsberg Biotechnology Ltd. A/S**
**Tagensvej 16**
**DK-2200 Copenhagen N(DK)**
Inventor: **Hansen, Soren**
**Tomsgaardsvej 102**
**DK-2400 Copenhagen NV(DK)**

(74) Representative: **Christiansen, Ejvind et al**
**HOFMAN-BANG & BOUTARD A/S**
**Adelgade 15**
**DK-1304 Copenhagen K (DK)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

The present invention concerns a process for enzymatic production of dipeptides and derivatives of dipeptides and having the general formula

H-A-B-Y

wherein A represents an optionally side-chain protected L-or D-α-amino acid residue or ω-amino acid residue and B represents an optionally side-chain protected L- or D- α-aminocarboxylic acid residue which may be the same as or different from A, and L- or D-aminophosphonic acid residue or L- or D-aminosulfonic acid residue or the corresponding ω-amino acids or salts and hydrates thereof, and Y is OH, H, alkyl, aryl, aralkyl or a C-terminal blocking group, with the proviso that A can not be Asp or Glu when B is Phe and Y is alkyl or BY represents an amino alcohol residue

$B^1$-$CHY^1$-OH

wherein $B^1$ is a decarboxy derivative of the aminocarboxylic acids as defined with relation to B, and $Y^1$ is H, alkyl, aryl or aralkyl.

In recent years there has been an increasing interest in dipeptides and dipeptide derivatives optionally containing an amino acid residue of D-configuration, with a view to their potential pharmacological effects, such as e.g. antibiotics. Likewise, there has been a great interest in dipeptides within fields such as artificial nutrition - human as well as veterinary -sweeteners and within agrochemistry, such as e.g. herbicides.

Such dipeptides H-A-B-Y can be produced by means of known chemical coupling reactions, but all these methods share the feature that, generally, it is necessary to protect the amino acids involved - A and B - on the amino group and the carboxylic acid group, respectively, and frequently also on the side chains if these carry functional groups. Further, there is an inherent risk of side reactions during the chemical coupling step because of the reagents and conditions employed, a major side reaction being recemization, particularly of the A-component. By replacing the chemical coupling step with an enzymatic coupling step proceeding under mild conditions, such side reactions and racemization can be avoided, yielding a stereochemically pure product.

The presence of amino- and carboxyl protective groups is mandatory in chemical coupling and has generally been regarded as mandatory also in prior art enzymatic coupling using endoproteases.

This adds several undesired features to these processes seriously afflicting their process economy on an industrial scale, particularly apparent in dipeptide synthesis.

The disadvantages are concerned with the introduction of these groups, as well as their removal and presence during process operation, increasing overall process cost and time and affecting overall yield.

Typical examples of amino protective groups commonly used are those of the carbobenzoxy (Z-) and tert-butoxycarbonyl (Boc-) type, which are of a molecular weight comparable to those of the amino acid residues. Firstly, the protective groups will have to be introduced in the starting materials by means of appropriate costly agents in a separate reaction step followed by an isolation step. While present, these hydrophobic groups often have a drastical effect upon the solubility of the intermediates and reaction products, and may afflict both the nature and the amount of solvents required in their processing as well as ease of purification and of deprotection. The deprotection will also take place in a separate step with a following purification step.

For this purpose a series of reactions are available, but with the exception of catalytical hydrogenation, posing industrial problems of its own, these methods are occuring under violent, often strongly acidic or basic conditions, frequently giving rise to a series of side reactions, resulting in an impure product or demanding laborious purification.

The last steps in this relatively long series of synthesis steps may thus be a rather comprehensive deprotection to obtain the desired peptides, and, owing to the almost inevitable secondary reactions, rather laborious purification procedures are frequently required to provide a product with the desired high purity.

A dipeptide which has attracted great attention in recent years is L-Asp-L-Phe-methylester, also known as aspartame, and derivatives thereof which has found extended use as sweeteners. The chemical synthesis of aspartame is entailed with the above-mentioned drawbacks.

Attempts to avoid amino terminal protection in the production of aspartame and its derivatives have led to microbial termentation approaches, like the fermentation processes described in EP-A1-074095, EP-A2-102529 and EP-A2-154472. This technique is fundamentally different from synthetic approaches and relies on specific organisms for aspartame, arid is thus not generally applicable in connection with other

dipeptides. In addition, the yields are low and recovery from the fermentation broth laborious.

The above-mentioned shortcomings in the known processes for the production of aspartame are confirmed in EP-A2-269390. In this application a method for producing L-Asp-L-Phe-alkylesters is claimed which comprises reacting in a solvent medium L-aspartic acid alpha ester or L-aspartic acid alpha amide with L-phenylalanine alkyl ester in the presence of an enzyme, microorganism containing the enzyme, enzyme containing fraction of a microorganism, or enzyme immobilized on solid support, said enzyme being capable of forming L-aspartyl-L-phenylalanine alkyl ester by condensation of the L-aspartic acid alpha ester or L-aspartic acid alpha amides and L-phenylalanine alkyl ester.

It is seen that an enzyme must be used which has specific esterase or amidase activity against Asp, but not agains Phe. The only enzyme mentioned is the extracellular protease with esterolytic activity of Staphylococcus aureus V8.

While therefore the applicability of N-$\alpha$-unprotected Asp-esters or amides is suggested - along with N-protected or $\beta$-esters or amides - the disclosure of one specific enzyme for the use in the production of a specific peptide supported only by one example where rather peculiar reaction conditions are used, viz. 5 times excess of substrate components, this reference does not by far provide a general teaching of the applicability of N-unprotected substrate components in dipeptide synthesis catalyzed by amidase or esterase enzymes.

Thus, it is an obvious advantage in terms of overall process economy to be able to avoid protective groups, also on the amino and carboxy terminus. In some cases, it may be of interest to be able to produce a dipeptide having side-chain protection, but no terminal protection, and it will be shown that this is possible in the process according to this invention, starting from side-chain protected, but amino unprotected starting materials. In this case, the same advantages of mild reaction conditions and overall process economy may be obtained. If desired, the side-chain protective group may be removed by chemical or enzymatic means, generally under milder conditions than amino protective groups.

The enzyme catalyzed coupling reactions enabling the use of optionally side-chain unprotected amino acid derivatives and an optionally C-terminal unprotected B-component (nucleophile) are known. See e.g. the DK Patent Specification No. 155613 as well as the corresponding EP Patent Specification No. 17 485 (EP-B1-17485), incorporated herein by reference.

Briefly, EP-B1-17485 describes a process for producing peptides by reacting a substrate component selected from i.a. amino acid esters and amino acid amides with an amine component (nucleophile) selected from i.a. L-amino acid amides, L-amino acids or L-amino acid esters in the presence of an L-specific serine or thiol carboxypeptidase enzyme.

If a dipeptide is to be produced by the process of EP-B1-17485, the substrate component is obligatory an N-terminal protected amino acid derivative, and the constituent amino acid is obligatory an L-amino acid.

As described in EP-A1-278787, (incorporated by reference) and other applications claiming priority from DK appln. 725/87 filed on February 13, 1987 it was surprisingly found that the serine and thiol carboxypeptidases used in EP-B1-17485 are capable of utilizing N-unprotected amino acid esters or amides as a substrate component in controlled reactions for synthesis of dipeptides and dipeptide derivatives, and that it was possible to suppress a possible oligomerization of the substrate.

Admittedly, it has long been known that some endoproteases can catalyze oligomerization of certain N-unprotected amino acid esters with L-configuration, (Fruton,-J.S. Advances in Enzymology, 53: 239-306, 1982) but it has never been attempted to use this for production of dipeptides which are not simple dimers. Generally, the results of such oligomerizations have been a mixture of a series of oligomers, sometimes long. The degree of oligomerisation and complexity of the mixture depend on the solubility of the products formed. Also it should be mentioned that the mere fact that a given enzyme can hydrolyze a particular N-$\alpha$ unprotected amino acid derivative does not automatically lead to a similar ability to catalyze coupling reactions involving the same derivative. See the references cited by Andersen, A.J. in "Peptides, Structure and Function". Proc. of the ninth ann. peptide symposium, Eds. Deber, C.M. et al., Pierce (1985), p. 355.

For this reason the use of serine and thiol endoproteases for peptide synthesis has been limited to the use of amino and carboxy terminal protected starting materials, as exemplified by US-A-4.086.136, where e.g. papain, stembromelein, ficin, chymopapain and chymotrypsin are mentioned.

Furthermore, for these enzymes free amino acids have so far generally been regarded as unsuitable as amino components as noted by Y.W. Mitin et al., Int. J. Peptide Protein Res., Vol. 23 (1984), p. 528-534.

The above-mentioned amino and carboxy terminal protected starting materials are also mandatory if aspartate endoproteases e.g. pepsin are used as exemplified by US-A-3.972.773, and if metallo endoproteases are used, as exemplified by the synthesis of Z-AspPheOMe.PheOMe-salt in EP-A1-009585. In these condensation type reactions it is further mandatory that the substrate component is on the free $\alpha$-carboxylic acid form, as exemplified in EP-A2-220923.

The enzymatic condensation of L-amino acids with α-aminophosphonic acids illustrated in EP-A2-209430 likewise makes use of an N-α-protected amino acid, e.g. BOC-Ala, BOC-Len or BOC-Phe, in the free carboxylic acid form as the substrate component.

The use of amino and carboxy terminal protected starting materials has also been considered mandatory for nonproteases, e.g. esterases with amidase activity, Blair West et al., Tetrahedron letters, Vol. 28. No. 15, p. 1629-32, (1987), who used porcine pancreatic lipase, Candida cylindracea lipase and pig liver esterase in organic media, optionally containing an aqueous buffer.

Cellulases in turn, have so far only been used synthetically for polymerisation condensation type reactions of completely free β-amino-acids as described by Kitazume, T. et al., J. Flourine Chem. 36 - (1987), p. 225-236.

The synthesis of the diastereomeric dipeptides of DL, LD and DD-configuration as well as peptides containing β-amino acid residues from amino-unprotected substrate components has so far not been possible with carboxypeptidases except as described with relation to EP-A1-278787 nor in general with any proteolytical enzymes (Class EC 3.4). Some efforts have been made with a different class of enzymes, aminoacyl-t-RNA-synthetase (Class EC 6.1) as exemplified by EP-A1-086053. In this case a specific enzyme must be used for each type of amino acid residue, and furthermore, expensive Co-factors like ATP are required. At the same time, yields are very poor, so even though some product was isolated and identified, typically a ten fold excess of Co-factor and a hundred fold excess of nucleophile and up to a thousand fold excess of enzyme by weight was required.

A recent study by Gaertner et al., Proteins: Structure, Function and Genetics 3: 130-137 (1988) indirectly confirms the prejudice against using N-α-unprotected amino acids as substrate components in enzymatic peptide synthesis. Since a major problem earlier encountered is secondary hydrolysis of the newly synthesized product, an attempt was made to decrease water activity in the reaction medium by using organic solvents. Gaertner et al. reports dipeptide synthesis using chymotrypsin which had been chemically modified to enhance its solubility in organic media. Using a number of N-α-protected amino acid esters as substrates and amino acid amides as nucleophiles N-α-protected dipeptide amides were obtained in good yields in a benzene medium. Thus, α-Bz-Lys-Phe-$NH_2$ was obtained in a yield of more than 98% by reacting α-Bz-Lys-OMe with Phe-NH2. However, when using ϵ-Z-LysOEt, i.e. a side-chain protected but N-α-unprotected lysine ester, no ϵ-Z-Lys-Phe-$NH_2$ was formed. Gaertner et al do not comment on this result, but it indicates that they apparently believed that amino group protection of the substrate component was compulsory.

The present invention which represents a further development of the invention according to EP-A1-278787 resides on the surprising finding that the capability of utilizing N-α-unprotected amino acid esters and amides as the substrate component in controlled reactions for synthesis of dipeptides and dipeptide derivatives is not limited to serine or thiol carboxypeptidases, but also possible with amidase or esterase enzymes generally, in particular serine endoproteases, thiol endoproteases, lipases and esterases.

Again, it was surprisingly found that also N-α-unprotected amino acid derivatives of D-configuration can be used as substrates in these reactions, so that, in addition to LL-dipeptides, it is also possible to synthesize DL-dipeptides. The reaction rate for D-substrates, however, is generally somewhat lower than for L-substrates under uniform conditions, but the difference in rate is much smaller than for the corresponding N-protected amino acid esters, the D-substrate being reacted at a rate which is much smaller than the rate for the L-substrate; Purdie et al., Biochem. Biophys. Acta, 268 (1972), 523. There are, however great individual differences between the enzymes in this respect. Synthesis yields are often just as high with the unprotected D-substrates in relation to the unprotected L-substrates.

It is known that certain endoproteases are capable of utilizing a nucleophile component having D-configuration in reactions with N-protected substrate components. Thus, depending on the actual sequence it is possible to synthesize dipeptides having LL,- DL-, LD- or DD-configuration by the process according to the invention.

The process of the invention is thus characterized by reacting a substrate component, which is an amino acid derivative having the formula

$H-A-OR^1$ or 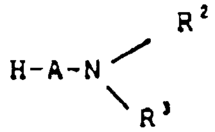

wherein A is as defined above, $R^1$ represents alkyl, aryl or aralkyl optionally substituted with inert substituents or an $\alpha$-des-amino fragment of an amino acid, and $R^2$ and $R^3$ are the same or different and each represents hydrogen, alkyl, aryl or aralkyl optionally substituted with inert substituents,
with a nucleophile component selected from

(a) amino acids having the formula

H-B-OH

wherein B is an aminocarboxylic acid residue as defined above,

(b) amino acid amides having the formula

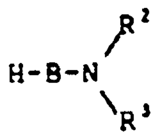

wherein B is an aminocarboxylic acid residue as defined above, and $R^2$ and $R^3$ have the above meaning, except that when $R^2$ represents hydrogen, $R^3$ may also represent hydroxy or amino.

(c) amino acid esters having the formula

$H\text{-}B\text{-}OR^4$

wherein B is an aminocarboxylic acid residue as defined above, and $R^4$ represents alkyl, aryl or aralkyl,

(d) optionally acid group protected straight chain or branched aminophosphonic acids or aminosulfonic acids having the formula

$NH_2(CH_2)_xPO_3R^5R^6$ or $NH_2(CH_2)_xSO_3R^7$

wherein $R^5$, $R^6$ and $R^7$ independently represent hydrogen, alkyl, aryl or aralkyl and x is 1-6,

(e) amino acid aldehydes or ketones or derivatives thereof having the formula

$H\text{-}B^1\text{-}CY^2\text{-}R^8$

wherein $B^1$ is as defined above, $Y^2$ is O or a functional derivative thereof, preferably a ketal, and $R^8$ is H, alkyl, aryl or aralkyl, and

(f) amino alcohols having the formula

$H\text{-}B^1\text{-}CHY^1\text{-}OH$

wherein $B^1$ and $Y^1$ have the above meaning
in the presence of an amidase or esterase enzyme different from serine or thiol carboxypeptidases in solution or dispersion, and then, if desired, cleaving a present side-chain protecting group or protective group Y and/or, if desired, converting the resulting dipeptide derivative to a salt or hydrate.

Examples of useful amino acids include aliphatic amino acids, such as monoaminomonocarboxylic acids, e.g. glycine (Gly), alanine (Ala), valine (Val), norvaline (Nval), leucine (leu), isoleucine (iso-Leu) and norleucine (Nleu), hydroxy amino acids, auch as serine (Ser), threonine (Thr) and homoserine (homo-Ser), sulfur-containing amino acids, such as methionine (Met) or cystine (CysS) and cysteine (CysH), monoaminodicarboxylic acids, such as aspartic acid (Asp), glutamic acid (Glu) and amides thereof, such as asparagine (Asn) and glutamine (Gln), diaminomonocarboxylic acids, such as ornithine (Orn) and lysine (Lys), arginine (Arg), aromatic amino acids, such as phenylalanine (Phe) and tyrosine (Tyr), as well as heterocyclic amino acids, such as histidine (His), proline (Pro) and tryptophan (Trp). As examples of useful amino compounds of a more unusual structure may be mentioned penicillamine (Pen), aminophosphonic acids, such as alanine-phosphonic acid (AlaP), aminosulfonic acids, such as taurine (Tau), $\omega$-amino acids, such as $\beta$-alanine (BAla), iso amino acids, such as $\alpha$-methylalanin (Aib), amino acids substituted with inert substituents, e.g. halogen or nitro, or the aldehyde, ketones, ketals or alcohols derived from e.g. alanine. As mentioned, they may be included in D-form in the substrate component and they may also be present in D-form in the nucleophile component.

It will be understood that the definitions given for the group Y in the peptide derivative of the formula H-A-B-Y are reflected in the various nucleophile components, stated in claim 1. Thus, (b) and (c) both contain C-terminal blocking groups, while Y = H corresponds to an amino acid aldehyde and Y = alkyl, aryl or aralkyl corresponds to an amino ketone. If desired a functional derivative of an amino ketone may be used as nucleophile, e.g. a ketal, oxime or sulfite.

As in the process according to EP-A1-278787, the advantages of the process of the present invention over the mentioned known methods are minimum or no side chain protection, no N-protection of the substrate component which may have both D- and L-configuration, no risk of racemization, few synthesis steps and an expected relatively pure end product, which in combination provides an extremely simple and economic method of production.

Preferred substrate components are esters in which $R^1$ is a straight or branched alkyl having 1 to 8 carbon atoms, such as methyl, ethyl, propyl, isopropyl, butyl, isobutyl, tert-butyl, amyl, hexyl, heptyl and octyl, or the aralkyl group benzyl. Particularly expedient nucleophile components are amino acid amides, in which $R^2$ is H, and $R^3$ is H or $C_1$-$C_6$ alkyl, or amino acid esters in which $R^4$ is a straight or branched alkyl having 1 to 6 carbon atoms such as the above-mentioned ones. As mentioned, $R^1$ may be alkyl, aryl or aralkyl optionally substituted with inert substituents, e.g. hydroxy or nitro.

The invention also comprises the processes involving intermediate formation of a peptide containing the group

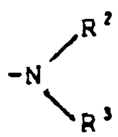 or $-OR^4$

following which this group may be cleaved to form a carboxylic acid group. This cleavage may be catalyzed by another enzyme or the same enzyme as was used to form the peptide, albeit under different reaction conditions. Also C-terminal modifications of the group Y may be made.

Enzymes may also be used to cleave side-chain protective groups, applicable enzymes being proteolytic enzymes, lipases and esterases, according to the nature of the protective group, see "The peptides, Analysis, Synthesis, Biology" Vol 9, Special Methods in peptide Synthesis Part C. J.A. Glass, Enzymatic manipulation of Protecting Groups in Peptide Synthesis, Academic Press 1987.

As examples of enzymes possessing esterase and/or amidase activity, and therefore expected to be active as catalysts in the process according to this invention may be mentioned those listed in the table below.

For a closer description of these enzymes, reference is made to i.a. Perlmann, G. E. et al., in Colowick, S. P. & Kaplan, N. O. (eds.) Methods of Enzym. 8 (1966) 19 (1970), 28 (1972), 35 (1975) and 45 (1976), Fruton, S., Adv. Enzymol. 53, p. 239, Wiley (1982), Abassi, A. et al. Biol. Chem. Hoppe-Seyler, 367, p. 441-45 (1986), Dixon, M. and Webb E. C. "Enzymes", 3rd ed. Longman Group (1979), Torrey, S. (ed.) in "Enzyme Technology, Recent Advances", Biotech, Rev. 2, Noyes Data Corporation (1983), and finally Laane, C., Tramper, S., Lilly, M. D. (eds.) "Biocatalysis in Organic Media", Elsevier (1987), Asano, Y. et al., Angew. Chem. 101, (1989), p. 511-512, Kitazume, T. et al., J. Fluorine Chem. 36 (1987), p. 225-236. all being incorporated by reference.

## TABEL I

### I. Proteases different from carboxypeptidases:

| | Enzyme | (Abb) | Normal Source |
|---|---|---|---|
| A) Thiolendo-proteases: | Papain | (P) | Papaya |
| | Chymopapain | (CP) | Papaya |
| | Bromelain | (B) | Pineapple |
| | Ficin | (F) | Fig (Ficus) |
| | Clostripain | (CL) | Clostridium histolyticum |
| B) Serineendo-proteases: | Trypsin | (T) | Pancreas |
| | Chymotrypsin | (CT) | Pancreas |
| | Elastase | (E) | Pancreas |
| | Subtilisin | (S) | Bacillus licheniformis or subtilis |
| | Thermitase | (TV) | Thermoactino-myces vulgaris |
| | Proteinase K | (K) | Tritirachium album |
| | Valyl-proteinase | (VP) | Candida tropicalis |
| | Post Prolin Specific Endopeptidase | (PPSE) | Flavo-bacterium meningo-septicum |

| | | |
|---|---|---|
| Achromobacter lyticus Protease I (AL-I) | | Achromobacter lyticus |
| Endoproteinase ArgC (AC) | | Submaxillaris glands |
| Endoproteinase LysC (LC) | | Lysobacter enzymogenes |
| Thrombin (TB) | | Blood plasma |

C) Exopeptidases:

| | | |
|---|---|---|
| Aminopeptidase (EC3.4.11) particularly from Achromobacter sp. | | Animal, vegetable, or microbial |

II. Other hydrolases acting on ester bonds

| | | |
|---|---|---|
| Carboxylesterase | (EC.3.1.1.1) | Liver |
| Arylhydrolase | (EC.3.1.1.2) | Plasma |
| Triacylglycerollipase particularly from Porcine Pancreas or Candida cylindracea or Lipolase® (NOVO); (Aspergillus sp.) | (EC.3.1.1.3) | Animal, vegetable, or microbial |
| Acetic ester acetylesterase | (EC.3.1.1.6) | Animal tissue |
| Arylglycerol lipase | (EC.3.1.1.23) | Animal or vegetable |

III Glycosidases

| | | |
|---|---|---|
| Cellulase particularly from Trichoderma viride or Aspergillus niger | (EC.3.2.1.4) | Animal, vegetable, or microbial |

The presently preferred enzymes are trypsin, chymotrypsin, subtilisin, elastase, papain, chymopapain, clostripain, porcine pancreatic lipase and Candida cylindracea lipase.

The enzyme used may also be chemically modified or be a biosynthetic mutant of a natural form.

As illustrated more fully below, the process of the invention is rather simple.

The reaction may be performed in an aqueous reaction medium, if desired containing up to 90%, preferably up to 60% of a polar organic solvent which is miscible with water, and compatible with the enzyme under the conditions specified. Preferred solvents are lower alcohols, dimethyl formamide, dimethyl

sulfoxide, dimethoxy ethane and ethylene glycol.

It is important to maintain a rather constant pH value in the reaction mixture. This pH value is between 3 and 11, preferably between 5 and 10.5, more preferably between 6 and 10 and most preferably between 7 and 9.5 and also depends upon the concrete starting materials, the peptide formed and the enzyme.

The reaction may alternatively be performed in a nonaqueous, nonpolar medium being composed of water immiscible organic solvents e.g. benzene, toluene, hexanes, heptanes, octanes, dialkyl ethers, ethyl acetate, ethyl propionate and methylene chloride, etc. which are compatible with enzymes having esterase and/or amidase activity, in particular lipases.

These essentially apolar reaction media may contain up to 10% water in dissolved form in order to facilitate optimal enzyme performance or reaction stability.

The reaction temperature is preferably room temperature and above, 20 to 50 °C, but temperatures in the range 0 to 80 °C may be used, if advantageous under the conditions otherwise given. At high solvent conditions subzero or temperatures higher than 80 °C may be used.

The concentration of the two reaction components may vary within wide limits, but the nucleophile component is frequently in excess, and to avoid oligomerization of the substrate component, said component is often added in minor portions at intervals during the entire reaction sequence. If a good nucleophile for the enzyme is used in high excess, surprisingly no oligomerization is observed, and high substrate concentrations may be used without side reactions. In case a homo-dipeptide in which A = B is wanted, oligomerization from a single nucleophile can be avoided by using different carboxy protective groups on the substrate and nucleophile component.

Thus, the starting concentration of the substrate component may typically be 0.005 to 2 molar and for the nucleophile component in cases, where it is added separately, 0.005 to 3 molar. In most situations it is possible to recover excess of the nucleophile component and the hydrolysis product from the substrate component for optional reesterification and reuse. Recycling of the components is particularly easy because of their simple structure and the absence of side reactions and deprotective losses.

The enzyme concentration may likewise vary, but is frequently somewhat higher (5-50 µm) (or even higher, up to ~ 500 µm) than the concentrations appropriate in the use of N-protected amino acid ester substrates, but the amount required for synthetic purposes may be reduced more than tenfold by using a stable immobilized enzyme preparation, thereby enabling the enzyme to be used in a continuous process.

The reaction medium may also contain salts, such as NaCl and $CaCl_2$, which influences the binding of the enzyme to the substrate and may stabilize the enzyme, as well as a complex binding agent for present metal ions, such as EDTA, and mercaptostabilizing agents such as DTT, BME or Cysteine may used with for instance thiolendoproteases.

This surprising hydrolysis sequence is reflected in the following examples, which illustrate the production of various dipeptides in the process of the invention using various enzymes.

<u>General method for examples 1-13 and 20 et seq.</u>

The reactions, performed on an analytical scale with a reaction volume of 1 ml, were carried out in a pH-stat, and the selected pH value was kept constant by automatic addition of 1 N NaOH except for the examples with high content of organic solvents or immobilized enzymes, where the conditions are indicated in the individual examples. Reaction temperature was room temperature, unless otherwise stated. The table also includes reaction concentrations, content of organic solvent, product and yield. Reaction times are typically between 0.5 and 5 hours and the enzyme concentrations are typically 5-20 µm. unless otherwise stated.

Product identification and determination of product yield were performed by means of reverse phase HPLC (Waters 6000 A pumps, 660 gradient blender, UK 6 injector) on a $C_{18}$NOVA PAK column (Waters, RCM) using suitable gradients of elution systems containing 50 mM triethylammonium phosphate, pH 3.0 from 0% to 80% acetonitrile with a flow of 2 ml/min. Elution was monitored by means of a UV detector (Waters 480) at 230 nm, 254 nm, 278 nm or 290 nm.

The products were identified by amino acid analysis of fractions from the HPLC analysis, which corresponded to the assumed product peak and/or by HPLC comparison with a chemically synthesized reference product. These were produced according to known principles, usually via reaction between BOC-A-OSu - the tertiary butyloxy carbonyl - succinic imide ester derivative of the substrate amino acid - and the used nucleophile component followed by deblocking of the N-terminal amino acid residue. In all cases, it was possible to separate LL-and DD-dipeptides from the diasteromeric DL-and LD-dipeptide products.

For the products which can only be detected at 230 nm, the product yields were determined by means of the absorption/concentration curve of the chemically synthesized reference compound. For the other

products, the yields were determined on the basis of the ratio between the integrated areas below the peaks in the elution chromatogram, corresponding to the product respectively the reactant which absorbs at the wavelength concerned.

The reaction conditions in the preparative examples 14-17 are described in the individual examples. The reactions were followed on analytical HPLC as described. The enzyme concentrations are generally lower and the reaction times longer than in the corresponding analytical examples, but no attempt to optimize the reaction conditions has been made.

Example 1

Trypsin [a]) catalyzed synthesis of L,L-dipeptide amides with L-Arginine ethyl ester (50 mM) as substrate and L-amino acid amides as nucleophiles at various concentrations in water at pH 8.5

| Nucleophile | (conc.) | Product | Yield |
|---|---|---|---|
| Leucine amide | (0.2 M) | $ArgLeuNH_2$ | 20% |
| Leucine amide | (0.7 M) | $ArgLeuNH_2$ | 32% |
| Methionine amide | (0.25 M) | $ArgMetNH_2$ | 31% |
| Methionine amide | (0.5 M) | $ArgMetNH_2$ | 52% |
| Methionine amide | (1.0 M) | $ArgMetNH_2$ | 90% |
| Serine amide | (0.5 M) | $ArgSerNH_2$ | 45% |
| Tyrosine amide | (0.5 M) | $ArgTyrNH_2$ | 46% |

[a]) 10 uM

Example 2

Trypsin [a]) catalyzed synthesis of diastereomeric L,D- and D,L-dipeptide amides with L- or D-Arginine ethyl ester (50 mM) as substrate and L- or D-Leucine and Methionine amides as nucleophiles in water at pH 8.5

| Substrate | Nucleophile | (conc.) | Product | Yield |
|---|---|---|---|---|
| D-argOEt | L-Leucine amide | (0.2 M) | $argLeuNH_2$ | 20% |
| D-argOEt | L-Leucine amide | (1.0 M) | $argLeuNH_2$ | 30% |
| D-argOEt | L-Methionine amide | (0.5 M) | $argMetNH_2$ | 68% |
| L-ArgOEt | D-methionine amide | (1.0 M) | $ArgmetNH_2$ | 5% |

[a]) 10 uM

Example 3

| Trypsin [a]) catalyzed synthesis of L,L-dipeptide amides using L-amino acid amides (0.5 M) as nucleophiles and L-Lysine or Histidine ethyl ester (50 mM) as substrate in water at pH 8.5 | | | |
|---|---|---|---|
| Substrate | Nucleophile | Product | Yield |
| LysOEt | Methionine amide | $LysMetNH_2$ | 81% |
| LysOEt | Tryptophane amide | $LysTrpNH_2$ | 32% |
| LysOEt | Alanine amide | $LysAlaNH_2$ | 5% |
| HisOEt | Methionine amide | $HisMetNH_2$ | 62% |

[a]) 10 uM

Example 4

| Alpha-Chymotrypsin [a]) catalyzed synthesis of L,L-dipeptide amides using L-Tyrosine ethyl ester (50 mM) as substrate and L-amino acid amides as nucleophiles in water | | | | |
|---|---|---|---|---|
| Nucleophile | (conc.) | pH | Product | Yield |
| Leucine amide | (0.2 M) | 9.0 | $TyrLeuNH_2$ | 68% |
| Arginine amide | (0.4 M) | 8.5 | $TyrArgNH_2$ | 90% |
| Serine amide | (0.4 M) | 8.5 | $TyrSerNH_2$ | 75% |

[a]) 5 uM

Example 5

| Alpha-Chymotrypsin [a]) catalyzed synthesis of L,D-dipeptide amides using L-Tyrosine ethyl ester (5 or 50 mM) as substrate and D-amino acid amides as nucleophiles in water | | | | |
|---|---|---|---|---|
| Nucleophile | (conc.) | pH | Product | Yield |
| D-leucine amide | (0.2 M) | 9.0 | $TyrLeuNH_2$ | 17%[b]) |
| D-isoleucine amide | (0.3 M) | 9.0 | $TyrileNH_2$ | 23%[b]) |
| D-serine amide | (0.4 M) | 8.5 | $TyrserNH_2$ | 35% |

[a]) 5 uM
[b]) 5 mM substrate

Example 6

| Alpha-Chymotrypsin catalyzed synthesis of D,L-dipeptide amides using D-tyrosine ethyl ester (50 mM) as substrate and L-Leucine amide as nucleophiles in water at pH 9.0 | | | |
|---|---|---|---|
| Nucleophile | (conc.) | Product | Yield[c]) |
| L-Leucine amide | (0.2 M) | D,L-tyrLeu$NH_2$ | 40%[a]) |
| L-Leucine amide | (0.3 M) | D,L-tyrLeu$NH_2$ | 68%[b]) |

[a]) 50 uM enzyme
[b]) 100 uM enzyme
[c]) Prolonged reaction time - days

Example 7

| Alpha-Chymotrypsin [a]) catalyzed synthesis of L,L- and L,D-dipeptide amides and esters using different L-amino acid esters (50 mM) as substrates and L- or D-amino acid esters (0.8 M) or amides as nucleophiles at pH 8.5 | | | | |
|---|---|---|---|---|
| Substrate | Nucleophile | Solvent | Product | Yield[d]) |
| L-PheOEt | L-Arg$NH_2$ [b]) | Water | PheArg$NH_2$ | 82% |
| L-TyrOEt | L-SerOEt | Water | TyrSerOEt | 48%[c]) |
| L-TyrOBzl | L-SerOEt | 30% DMF | TyrSerOEt | 40%[c]) |
| L-TyrOBzl | L-SerOMe | 30% DMF | TyrSerOMe | 39%[c]) |
| L-TyrOBzl | D-serOMe | 30% DMF | TyrserOMe | 21%[c]) |

[a]) 5 uM
[b]) 0.4 M nucleophile
[c]) at 90% conversion
[d]) hydrolysis/diketopiperazine formed due to chemical instability of product was observed under these conditions in amounts of 15-35% and is not included in the yields reported.

Example 8

| Subtilisin A [a]) catalyzed synthesis of sidechain protected L-Aspartyl-D-alanyl amide using L-Aspartyl diesters (0.1 M) as substrates and D-alanine amide as nucleophile at pH 8.5 | | | | |
|---|---|---|---|---|
| Substrate | Nucleophile (conc.) | Solvent | Product | Yield |
| L-Asp$(OEt)_2$ | (1.0 M) | Water | Asp(OEt)ala$NH_2$ | 9% |
| L-Asp$(OEt)_2$ | (2.0 M) | Water | Asp(OEt)ala$NH_2$ | 24% |
| L-Asp$(OBzl)_2$ [b]) | (0.5 M) | 30% DMSO | Asp(OBzl)ala$NH_2$ | 8% |
| L-Asp$(OBzl)_2$ [b]) | (1.0 M) | 30% DMSO | Asp(OBzl)ala$NH_2$ | 20% |

[a]) 5 uM
[b]) 50 mM substrate, 20 uM enzyme

Example 9

| Elastase [a]) catalyzed synthesis of L,L-amides using amino acid benzyl ester (50 mM) as substrate and amino acid amide (0.5 M) as nucleophile in water at pH 8.5 | | | |
|---|---|---|---|
| Substrate | Nucleophile | Product | Yield |
| L-ValOBzl | L-Arginine amide | $ValArgNH_2$ | 59% |

[a]) 40 uM

Example 10

| Synthesis of L,L-dipeptide amides catalyzed by papaya thiolendoproteases[a]) using amino acid esters (50 mM) as substrates and L-amino acid amides (0.8 M) as nucleophiles in water at pH 8.5 | | | | |
|---|---|---|---|---|
| Enzyme | Substrate | Nucleophile | Product | Yield |
| Papain | LysOEt | $AlaNH_2$ | $LysAlaNH_2$ | 60% |
| Papain | LysOMe | $AlaNH_2$ | $LysAlaNH_2$ | 55% |
| Chymopapain | LysOEt | $AlaNH_2$ | $LysAlaNH_2$ | 23% |
| Chymopapain | LysOMe | $AlaNH_2$ | $LysAlaNH_2$ | 34% |

[a]) 100 uM, 2mM EDTA, 10 mM Cysteine

Example 11

| Clostripain [a]) catalyzed synthesis of L,L-dipeptide amides and esters with L-Arginine ethyl ester as substrate (50 mM) and L-amino acid amides as nucleophiles at pH 8.5 | | | | |
|---|---|---|---|---|
| Nucleophile | (conc.) | Solvent | Product | Yield |
| L-Methionine amide | (0.5 M) | Water | $ArgMetNH_2$ | 38% |
| L-Phenylalanine amide | (0.2 M) | 30% EtOH | $ArgPheNH_2$ | 6% |
| L-Phenylalanine amide | (0.6 M) | 30% DMF | $ArgPheNH_2$ | 65% |

[a]) 5 uM enzyme, 50 mM $CaCl_2$, 10 mM DTT, pH adjusted with TEA

Example 12

| Porcine Pancrease Lipase [a]) catalyzed synthesis of L,L-dipeptide amides using L-amino acid ethyl esters (50 mM) as substrates and L-amino acid amides as nucleophiles in 30% EtOH at pH 8.5 | | | | |
|---|---|---|---|---|
| Substrate | Nucleophile | (conc.) | Product | Yield |
| L-TrpOEt | L-Met$NH_2$ | (1.5 M) | TrpMet$NH_2$ | 71% |
| L-TrpOEt | L-Met$NH_2$ | (1.0 M) | TrpMet$NH_2$ | 54% |
| L-TyrOEt | L-Ser$NH_2$ | (1.5 M) | TyrSer$NH_2$ | 69% |
| L-MetOEt | L-Met$NH_2$ [b]) | (1.0 M) | MetMet$NH_2$ | 31%[c]) |

[a]) 500 uM
[b]) Reaction in pure water
[c]) At incomplete conversion

Example 13

| Candida Cylindracea Lipase [a]) catalyzed synthesis of L,L-dipeptide amides using L-amino acid ethyl esters (50 mM) as substrates and L-amino acid amides as nucleophiles in 30% EtOH at pH 8.5 | | | | |
|---|---|---|---|---|
| Substrate | Nucleophile | (conc.) | Product | Yield |
| L-TrpOEt | L-Met$NH_2$ | (1.5 M) | TrpMet$NH_2$ | 75%[b]) |
| L-TyrOEt | L-Ser$NH_2$ | (1.5 M) | TyrSer$NH_2$ | 50%[b]) |

[a]) 1000 uM, prolonged reaction time
[b]) Versus hydrolysis at less than 50% conversion

Example 14

Preparative synthesis of L,L-TryptophanylMethionine amide, TrpMet$NH_2$

Procedure

L-Tryptophane ethyl ester hydrochloride (4.0 g, 15 mmol) and L-Methionine amide hydrochloride (55.7 g, 300 mmol) were dissolved in 195 ml $H_2O$ and 90 ml ethanol, and pH was adjusted to 8.5 with sodium hydroxide. The reaction was initiated by addition of 0.8 g of crude Porcine Pancreatic Lipase and was kept at pH 8.5 for the duration of the reaction. The remainder of the substrate (4.0 g, 15 mmol) was added after 5 hours and the reaction continued overnight. It was then stopped by adjusting pH to 3 with HCl-solution.

The mixture was then diluted, ethanol was removed by evaporation under reduced pressure, and the mixture was filtered. The filtrate was purified by RP-preparative HPLC (Waters Prep LC/System 500A) using two columns (5.7 x 30 cm) packed with 60 um C-18 particles and 5 mM HCl/ethanol mixtures as eluent.

Collected fractions containing pure product were concentrated under reduced pressure and finally freeze dried.

This procedure gave 4.78 g of L,L-Tryptophanylmethionine amide hydrochloride (12.9 mmol, 43%) as an amorphous powder.

Identification

The product was identified as the hydrochloride containing 10.3% (w/w) of chloride.

Amino acid analysis showed the absence of free amino acids and gave the following results after acid hydrolysis:
Met (1.00)
Trp (1.00)

Specific optical rotation in 50% MeOH, c = 0.2 using the sodium D-line was found to be +40.0° at 20°C.

Purity

HPLC-purity: 92.4% (Novapak 4 um C-18, 0.1 M ammonium phosphate, pH 3.0/acetonitrile, 220 nm)
Water content by Karl Fisher: 5.3% (w/w)
Quantization of the alpha-amino group by reaction with Trinitrobenzene sulphonic acid and UV-detection: 75.8% (w/w).
Peptide content by UV-quantization: 88.3% (w/w) (281 nm, Trp-absorbance in MeOH: 0.1 N KOH (1:1))

Example 15

Preparative synthesis of L,D-Tyrosylserin amide, $TyrserNH_2$

Procedure

L-Tyrosine ethyl ester hydrochloride (2.5 g, 10 mmol) and D-serine amide hydrochloride (17.5 g, 100 mmol) were dissolved in 200 ml of water, and pH was adjusted to 8.5 with sodium hydroxide. The reaction was initiated by addition of 50 mg of alpha-chymotrypsin and was kept at pH 8.5 for the duration of the reaction. After 30 minutes precipitation of free tyrosine began. The remainder of the substrate (5.0 g, 20 mmol) was added in two portions of 2.5 g during 1 hour. The reaction was stirred for two hours and was then stopped by adjusting pH to 3 with HCl-solution.

The formed tyrosine was filtered off, and the filtrate was purified by RP-preparative HPLC (Waters Prep LC/System 500A) using two columns (5.7 x 30 cm) packed with 20 uM C-18 particles and 50 uM acetic acid as an eluent.

Collected fractions containing pure product were concentrated under reduced pressure and finally freeze dried with addition of aqueous HCl.

This procedure gave 2.8 g of L,D-Tyrosylserine amide hydrochloride (9.2 mmol, 31%) as an amorphous powder.

Identification

The product was identified as the hydrochloride containing 16.0% (w/w) of chloride.

Amino acid analysis showed the absence of free amino acids, but following acid hydrolysis gave the following results:
Ser (1.10)
Tyr (0.90)
Specific optical rotation in 50% MeOH, c = 0.3 using the sodium D-line was found to be +58.0° at 20°C.

Purity

HPLC-purity: 97.4% (Novapak 4 um C-18, 0.1 M ammonium phosphate, pH 3.0/acetonitrile, 220 nm)
Water content by Karl Fisher: 1.8% (w/w).
Quantization of the alpha-amino group by reaction with Trinitrobenzene sulphonic acid and UV-detection: 81% (w/w).

Example 16

Preparative synthesis of D,L-tyrosylLeucin amide, $tyrLeuNH_2$

Procedure

D-tyrosine ethyl ester hydrochloride (3.5 g, 14 mmol) and L-Leucin amide hydrochloride (14 g, 84 mmol) were dissolved in 246 ml of 0.1 M KCl and pH was adjusted to 9.0 with sodium hydroxide. The reaction was initiated by addition of 0.7 g of alpha-chymotrypsin and stirred for two days at room temperature. pH was kept at 9.0 for the duration of the reaction. The reaction was stopped by adjusting pH to 3 using HCl-solution.

The formed tyrosine was filtered off, and the filtrate was purified by RP-preparative HPLC (Waters Prep LC/System 500A) using two columns (5.7 x 30 cm) packed with 20 uM C-18 particles and 5 mM HCl as an eluent.

Collected fractions containing pure product were concentrated by evaporation under reduced pressure and finally freeze dried.

This procedure gave 2.50 g of D,L-tyrosylLeucine amide hydrochloride (7.6 mmol, 54%) as an amorphous powder.

Identification

The product was identified as the hydrochloride containing 9.8% (w/w) of chloride.

Amino acid analysis showed the absence of free amino acids, but following acid hydrolysis gave the following results:
Tyr (0.98)
Leu (1.03)
Specific optical rotation in water, c = 0.1 using the sodium D-line was found to be -129.4° at 20°C.

Purity

HPLC-purity: 92.9% (Novapak 4 um C-18, 0.1 M ammonium phosphate, pH 3.0/acetonitrile, 220 nm)
Water content by Karl Fisher: 6.8% (w/w).

Quantization of the alpha-amino group by reaction with Trinitrobenzen sulfonic acid and UV-detection: 74.9%
UV-quantization: 74.9% (Tyrosine phenolate absorbance at 293 nm, in 0.1 M KOH)

Example 17

Preparative synthesis of L,L-ArginylMethionine amide, Arg-MetNH$_2$

Procedure

L-Arginine ethyl ester dihydrochloride (4.1 g, 15 mmol) and L-Methionine amide hydrochloride (55.4 g, 300 mmol) were dissolved in 300 ml of water, and pH was adjusted to 8.5 with sodium hydroxide. The reaction was initiated by addition of 50 mg of trypsin. pH was kept at 8.5 for the duration of the reaction. The remainder of the substrate (8.2 g, 30 mmol) was added during one hour. The reaction was then stopped by adjusting pH to 3 using HCl-solution.

The reaction mixture was then diluted and purified by successive cation exchange on a DOWEX A650 Wx4 and a CM-Sepharose 6B column using ammonium acetate and NaCl/HCl salt gradients, respectively, and was finally desalted.

Collected fractions containing pure product were concentrated under reduced pressure and finally freeze dried.

This procedure gave 10.7 g of L,L-ArginylMethionine amide dihydrochloride (28.3 mmol, 63%) as a white amorphous powder.

Identification

Less than 0.2% (w/w) of acetate and 22.9% (w/w) of chloride were measured, so the product was present as a dihydrochloride.

Amino acid analysis showed the absence of free amino acids, but following acid hydrolysis gave the following results:
Arg (1.00)
Met (0.80)
Specific optical rotation in 50% MeOH, c = 0.2 using the sodium D-line was found to be +19.5° at 20°C.

Purity

HPLC-purity: 95.1% (Novapak C-18, 0.1 M ammonium phosphate containing alkylsulfonate, pH 4.5/acetonitrile, 220 nm)

Water content by Karl Fisher: 9.1% (w/w).

Peptide content by amino acid analysis: 72% (w/w) based on Arginine.

Example 18

| Synthesis of L,L-Methionyl-Methionine amide catalyzed by Eupergit C immobilized Porcine Pancreatic Lipase [a]) using L-Methionine ethyl ester as substrate and L-Methionine amide as nucleophile at pH 8.5 in water and water/organic homogeneous mixtures. | | | | |
|---|---|---|---|---|
| Conc. of substrate | Nucleophile | % | Organic solvent | Yield [b]) |
| 50 mM | 1.0 M | 0 | - | 68% |
| 100 mM | 0.5 M | 30 | Isopropanol | 26% |

[a]) Porcine Pancreatic Lipase was immobilized on Eupergit C using the procedure recommended by the manufacturer to a final concentration corresponding to approx. 400 µM on the gel by activity assay.

[b]) The reaction mixtures with volumes of 1-3 reaction bed volumes were recirculated over the column packed with enzyme gel until full conversion of the substrate as determined by HPLC (0.5-2 days) while pH was being kept constant by pH-stat control.

Example 19

| Porcine Pancreatic Lipase [a]) and alpha-chymotrypsin [a]) catalyzed synthesis of L,L-Tryptophanyl-Alanine-tert-butylester in pure organic solvents with L-Tryptophane ethyl ester (50 mM) as substrate and L-Alanine-tert-butylester (0.3 M) as nucleophile, both in saltfree form. | | | |
|---|---|---|---|
| Enzyme [a]) | Solvent | Conversion [c]) | Yield [c]) |
| PPL | $CH_2Cl_2$/n-Hexane (1:3) | 30% | 13% |
| CT | $CH_2Cl_2$/n-Hexane (1:3) | 56% | 17% |
| PPL | $CH_2Cl_2$ | 7% | 10% |
| CT | $CH_2Cl_2$ | 50% | 3% |
| PPL [b]) | $CH_2Cl_2$/Isooctane (1:1) | 82% [b]) | 26% |

[a]) 500-2000 µM freezedried enzyme containing moisture was added directly to the mixture, which was stirred for 24 hours.

[b]) In this case, 500 µM enzyme and 3 days of reaction time were applied.

[c]) Conversion of starting material and yield versus hydrolysis was determined by HPLC of samples quenched with DMF, evaporated and redissolved in DMF/acid water. Controls showed neither conversion nor yield.

Example 20

| Alpha-chymotrypsin [a]) catalyzed synthesis of sidechain protected L,L-dipeptide amides with L-Tyrosine or L-Phenylalanine ethyl esters (50 mM) as substrates and L-S-Acetamidomethylcystein amide (0.6 M) as nucleophiles in water at pH 8.5. | | |
|---|---|---|
| Substrate | Product | Yield |
| L-Tyrosine ethyl ester | TyrCys(-SAcm)$NH_2$ | 62% |
| L-Phenylalanine ethyl ester | PheCys(-SAcm)$NH_2$ | 78% |

[a]) 5 µM

Example 21

| Alpha-chymotrypsin [a]) catalyzed synthesis of L,L-Dipeptide alcohols using L-Tyrosine and L-Phenylalanine ethyl esters (50 mM) as substrates and L-Amino acid alcohols (0.5 M) as nucleophiles in water at pH 8.5. | | | |
|---|---|---|---|
| Substrate | Nucleophile | Product | Yield |
| L-TyrOEt | L-Met$CH_2$OH | TyrMet$CH_2$OH | 42% |
| L-TyrOEt | L-Leu$CH_2$OH | TyrLeu$CH_2$OH | 46% |
| L-PheOEt | L-Met$CH_2$OH | PheMet$CH_2$OH | 60% |

[a]) 10 µM

Example 22

| Synthesis of L,L-dipeptides catalyzed by thiolendoproteases [a]) using amino acid ethyl esters (50 mM) as substrate and free L-Amino acids as nucleophiles in water at pH 8.5. | | | | | |
|---|---|---|---|---|---|
| Enzyme | Substrate | Nucleophile | (conc.) | Product | Yield [b])[c]) |
| Ficin | ArgOEt | ArgOH | (1.0 M) | ArgArgOH | 6% |
| Papain | ArgOEt | ArgOH | (1.0 M) | ArgArgOH | 7% |
| Ficin | LysOEt | AlaOH | (1.5 M) | LysAlaOH | 5% |
| Papain | LysOEt | AlaOH | (1.5 M) | LysAlaOH | 6% |

[a]) 100 µM, 2 mM EDTA, 0.1 M KCl, 5 mM DTT or 10 mM cysteine.
[b]) Determined vs. hydrolysis via a standard at less than 50% conversion.
[c]) Controls without enzyme showed no detectable aminolysis under the conditions reported.

Example 23

| Trypsin [a]) catalyzed synthesis of L,L-dipeptide amides with Arginine-paranitroanilide (10 mM) as substrate and L-Amino acid amides (0.3 M) as nucleophiles in 40% DMF at pH 8.5. | | |
|---|---|---|
| Nucleophile | Product | Yield [b]) |
| Methionine amide | $ArgMetNH_2$ | 45% |
| Leucine amide | $ArgLeuNH_2$ | 31% |
| Tyrosine amide | $ArgTyrNH_2$ | 14% |

[a]) 5 µM

[b]) Determined vs. hydrolysis via a standard at less than 80% conversion.

Example 24

| Papain [a])[b]) catalyzed synthesis of L,L-Lysyl-Alanine amide using L-Lysine ethyl ester (50 mM) as substrate and L-Alanine amide (0.8 M) as nucleophile in water at various pH-values. | |
|---|---|
| pH | Yield [c]) |
| 6.5 | 21% |
| 4.5 | 47% |

[a]) 50 µM, 2 mM EDTA, 10 mM Cysteine.

[b]) At prolonged reaction time; less than 50% conversion.

[c]) Determined vs. hydrolysis using a standard and corrected for incomplete conversion.

Example 25

| Alpha-chymotrypsin [a]) and clostripain [b]) catalyzed synthesis of L,L-dipeptide esters using L-Amino acid ethyl esters (50 mM) as substrates and L-Amino acid ethyl or tert-butyl-esters as nucleophiles in water at pH 7.5 and 8.5. | | | | | |
|---|---|---|---|---|---|
| Enzyme | Substrate | Nucloephile | (conc.) | Product | Yield |
| CT | TrpOEt | AlaOtBu | (0.8 M) | TrpAlaOtBu | 12% |
| CT | TrpOEt | ValOEt | (0.8 M) | TrpValOEt | 18% |
| CL | ArgOEt | MetOEt | (1.0 M) | ArgMetOEt | 33% [c]) |

[a]) 5 µM, 0.1 M KCl, pH 7.5.

[b]) 10 µM, 50 mM $CaCl_2$, 10 mM DTT, pH 8.5 adjusted with TEA.

[c]) Determined at less than 50% conversion.

Example 26

Porcine Pancreatic Lipase [a]) and Lipolase (Novo)(LIP) [a]) and Rhizopus Arrhizus Lipase (RA) [b]) catalyzed synthesis of L,L-Methionyl-Methionine amide using different L-Methionine esters as substrates and L-Methionine amide as nucleophile in various aqueous/organic solvent homogeneous mixtures at pH 8.5 and 1.0 M nucleophile, unles otherwise indicated.

| Enzyme | Ester substrate | (conc.) | % | Organic solvent | Yield [e]) |
|---|---|---|---|---|---|
| PPL [c]) | Ethyl | (50 mM) | 0 | - | 55% |
| PPL [d]) | Ethyl | (50 mM) | 0 | - | 36% |
| PPL | Ethyl | (150 mM) | 0 | - | 42% |
| PPL | Ethyl | (200 mM) | 15 | Dimethoxy Ethane | 20% |
| PPL | Ethyl | (150 mM) | 30 | Isopropanol | 36% |
| PPL | Ethyl | (50 mM) | 90 | Ethylene Glycol | 42% |
| PPL | n-Propyl | (50 mM) | 0 | - | 55% |
| PPL | n-Hexyl | (50 mM) | 0 | - | 39% |
| LIP | Ethyl | (50 mM) | 0 | - | 47% |
| LIP | Ethyl | (50 mM) | 15 | Dimethoxy Ethane | 35% |
| LIP | n-Hexyl | (50 mM) | 0 | - | 32% |
| RA | Ethyl | (50 mM) | 30 | Ethanol | 23% |

a) 1000 μM - 2000 μM

b) 50 μM

c) pH 9.0

d) At 0.25 M nucleophile

e) Various minor amounts of deamidation of product was noted under these conditions and is not included in the yields reported.

Example 27

| Trichoderma Viridae Cellulase catalyzed synthesis of L,L-$TyrGlyNH_2$ using L-Tyrosine ethyl ester as a substrate and Glycine amide as nucleophile in water. | | | | | |
|---|---|---|---|---|---|
| Substrate | (conc.) | Nucleophile | (conc.) | Product | Yield |
| TyrOEt [a]) | (20 mM) | $GlyNH_2$ | (0.6 M) | $TyrGlyNH_2$ | 23% [c]) |
| TyrOEt [b]) | (10 mM) | $GlyNH_2$ | (0.8 M) | $TyrGlyNH_2$ | 34% [c]) |
| TyrOiPr [b]) | (10 mM) | $AlaNH_2$ | (1.5 M) | $TyrAlaNH_2$ | 52% [d]) |

[a]) 1000 µM crude enzyme, pH 8.5
[b]) 500 µM crude enzyme, pH 8.0
[c]) Determined vs. hydrolysis at less than 30% conversion and corrected for hydrolysis and aminolysis found in controls at similar conditions.
[d]) Complete enzymatical and no spontaneous conversion was observed under the conditions employed

## Claims

**1.** A process for producing dipeptides or structurally related compounds having the general formula

H-A-B-Y

wherein A represents an optionally side-chain protected L-or D-α-amino acid residue or ω-amino acid residue and B represents an optionally side-chain protected L- or D-α-aminocarboxylic acid residue which may be the same as or different from A, an L- or D-aminophosphonic acid residue or L- or D-aminosulfonic acid residue or the corresponding ω-amino acids or salts and hydrates thereof, and Y is OH, H, alkyl, aryl, aralkyl or a C-terminal blocking group, with the proviso that A can not be Asp or Glu when B is Phe and Y is alkyl or BY represents an amino alcohol residue

$B^1$-$CHY^1$-OH

wherein $B^1$ is a decarboxy derivative of the aminocarboxylic acids as defined with relation to B, and $Y^1$ is H, alkyl, aryl or aralkyl, **characterized** by reacting a substrate component, which is an amino acid derivative having the formula

$H-A-OR^1$ or 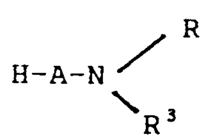

wherein A is as defined above, $R^1$ represents alkyl, aryl or aralkyl optionally substituted with inert substituents or an α-des-amino fragment of an amino acid, and $R^2$ and $R^3$are the same or different and each represents hydrogen, alkyl, aryl or aralkyl optionally substituted with inert substituents,
with a nucleophile component selected from
(a) amino acids having the formula

H-B-OH

wherein B is an aminocarboxylic acid residue as defined above,

(b) amino acid amides having the formula

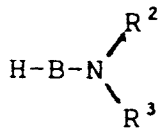

wherein B is an aminocarboxylic acid residue as defined above, and $R^2$ and $R^3$ have the above meaning, except that when $R^2$ represents hydrogen, $R^3$ may also represent hydroxy or amino,
(c) amino acid esters having the formula

$H\text{-}B\text{-}OR^4$

wherein B is an aminocarboxylic acid residue as defined above, and $R^4$ represents alkyl, aryl or aralkyl,
(d) optionally acid group protected straight chain or branched aminophosphonic acids or aminosulfonic acids having the formula

$NH_2(CH_2)_xPO_3R^5R^6$ or $NH_2(CH_2)_xSO_3R^7$

wherein $R^5$, $R^6$ and $R^7$ independently represent hydrogen, alkyl, aryl or aralkyl and x is 1-6
(e) amino acid aldehydes or ketones or derivatives thereof having the formula

$H\text{-}B^1\text{-}CY^2\text{-}R^8$

wherein $B^1$ is as defined above, $Y^2$ is O or a functional derivative thereof, preferably a ketal, and $R^8$ is H, alkyl, aryl or aralkyl, and
(f) amino alcohols having the formula

$H\text{-}B^1\text{-}CHY^1\text{-}OH$

wherein $B^1$ and $Y^1$ have the above meaning
in the presence of an amidase or esterase enzyme different from serine or thiol carboxypeptidases in solution or dispersion, and then, if desired, cleaving a present side-chain protecting group or protective group Y and/or, if desired, converting the resulting dipeptide derivative to a salt or hydrate.

**2.** A process according to claim 1, **characterized** by using an enzyme with esterase or amidase activity selected from serine or thiolendoproteases, lipases, esterases and glycosidases.

**3.** A process according to claim 1 or 2, **characterized** in that the enzyme used has been chemically modified or is a biosynthetic mutant of a natural form.

**4.** A process according to any of the preceding claims, **characterized** by using an immobilized enzyme.

**5.** A process according to any of the preceding claims, **characterized** by using an aqueous reaction solution or dispersion containing 0-90%, preferably 0-60%, of a polar water miscible organic solvent and having a pH value of 3-11, preferably 5-10.5, more preferably 6-10, in particular 7-9.5.

**6.** A process according to claim 5, **characterized** in that the organic solvent is selected from alkanols, dimethyl sulfoxide, dimethyl formamide, dimethoxy ethane and ethylene glycol.

**7.** A process according to any of claims 1 to 4, **characterized** by using an organic reaction solution or dispersion containing 0-10% of water.

**8.** A process according to claim 7, **characterized** by using an unpolar organic solvent, preferably selected from dialkyl ethers, ethyl acetate, ethyl propionate, octanes, heptanes, hexanes, petroleum

ether and methylene chloride.

**9.** A process according to claim 7, **characterized** by using a liquid substrate or nucleophile component which may also serve as the organic solvent.

**10.** A process according to any of the preceding claims, **characterized** by using as substrate component a D- or L-amino acid ester selected from benzyl esters or straight or branched $C_1$-$C_8$ alkyl esters optionally substituted with inert substituents.

**11.** A process according to any of the preceding claims, **characterized** by using as nucleophile component an amino acid amide having the formulae

H-B-NH$R^3$

wherein $R^3$ is hydrogen or $C_1$-$C_3$ alkyl and B is an L- or D-amino carboxylic acid residue.

**12.** A process according to any of claims 1 to 10, **characterized** by using as nucleophile component an ester having the formula

H-B-O$R^4$

wherein B is a L- or D-aminocarboxylic acid residue and $R^4$ is $C_1$-$C_3$ alkyl.

**13.** A process according to any of the preceding claims, **characterized** in that the resulting dipeptide includes one or more C-terminal protective groups Y, and that the group or groups are cleaved enzymatically, either by means of the same enzyme as was used in the preceding reaction or by means of an enzyme having a different ester or amide specifically.

**14.** A process according to any of the preceding claims, **characterized** in that the resulting dipeptide includes one or more side-chain protective groups and that the group or groups are cleaved enzymatically, preferably by means of an esterase or lipase or proteolytical enzyme.

**Patentansprüche**

**1.** Verfahren zur Herstellung von Dipeptiden oder strukturell verwandter Verbindungen der allgemeinen Formel

H-A-B-Y

in der A einen gegebenenfalls seitenkettengeschützten L-oder D-alpha-Aminosäurerest oder omega-Aminosäurerest und B einen gegebenenfalls seitenkettengeschützten L- oder D-alpha-Aminocarbonsäurerest, der gleich oder verschieden von A sein kann, einen L- oder D-Aminophosphonsäurerest oder einen L- oder D-Aminosulfonsäurerest oder die entsprechenden omega-Aminosäuren oder Salze oder Hydrate derselben und Y OH, H, Alkyl, Aryl, Aralkyl oder eine C-terminale Blockierungsgruppe bedeuten, mit der Maßgabe, daß A nicht Asp oder Glu sein kann, wenn B Phe und Y Alkyl ist, oder BY einen Aminoalkoholrest

$B^1$-CH$Y^1$-OH

bedeutet, wobei $B^1$ ein Decarboxyderivat der Aminocarbonsäure, wie bezüglich B definiert, und $Y^1$ H, Alkyl, Aryl oder Aralkyl ist, dadurch gekennzeichnet, daß man eine Substratkomponente, die ein Aminosäurederivat der Formel

H-A-O$R^1$ oder H-A-N$R^2R^3$

ist, wobei A wie oben definiert ist, sowie $R^1$ Alkyl, Aryl oder Aralkyl, gegebenenfalls mit inerten Substituenten, oder ein alpha-Desaminofragment einer Aminosäure und $R^2$ und $R^3$, die gleich oder verschieden sind, jeweils Wasserstoff, Alkyl, Aryl oder Aralkyl, gegebenenfalls substituiert mit inerten

Substituenten, bedeuten,
mit einer nucleophilen Komponente, ausgewählt aus

(a) Aminosäuren der Formel

H-B-OH

in der B ein wie oben definierter Aminocarbonsäurerest,

(b) Aminosäureamiden der Formel

$H\text{-}B\text{-}NR^2R^3$

in der B ein wie oben definierter Aminocarbonsäurerest ist und $R^2$ und $R^3$ wie oben definiert sind, mit der Ausnahme, daß wenn $R^2$ Wasserstoff ist, $R^3$ auch Hydroxy oder Amino sein kann,

(c) Aminosäureestern der Formel

$H\text{-}B\text{-}OR^4$

in der B ein wie oben definierter Aminocarbonsäurerest ist und $R^4$ Alkyl, Aryl oder Aralkyl bedeutet,

(d) gegebenenfalls säuregruppengeschützten, geradkettigen oder verzweigten Aminophosphonsäuren oder Aminosulfonsäuren der Formel

$NH^2(CH_2)_xPO_3R^5R^6$ oder $NH_2(CH_2)_xSO_3R^7$

in der $R^5$, $R^6$ und $R^7$ unabhängig voneinander Wasserstoff, Alkyl, Aryl oder Aralkyl und x 1 - 6 bedeuten,

(e) Aminosäurealdehyden oder Ketonen oder Derivaten derselben der Formel

$H\text{-}B^1\text{-}CY^2\text{-}R^8$

in der $B^1$ wie oben definiert, $Y^2$ O oder ein funktionelles Derivat desselben, vorzugsweise ein Ketal und $R^8$ H, Alkyl, Aryl oder Aralkyl bedeuten, und

(f) Aminoalkoholen der Formel

$H\text{-}B^1\text{-}CHY^1\text{-}OH$

in der $B^1$ und $Y^1$ wie oben definiert sind,

in Gegenwart eines Amidase- oder Esteraseenzyms, das von Serin- oder Thiolcarboxypeptidasen verschieden ist, in Lösung oder Dispersion umsetzt und anschließend, falls erwünscht, eine vorhandene Seitenkettenschutzgruppe oder Schutzgruppe Y abspaltet und/oder, falls erwünscht, das erhaltene Dipeptidderivat in ein Salz oder Hydrat überführt.

**2.** Verfahren nach Anspruch 1, dadurch gekennzeichnet, daß man ein Enzym mit Esterase- oder Amidase-Aktivität verwendet, das von Serin- oder Thiolendoprotheasen, Lipasen, Esterasen und Glycosidasen ausgewählt ist.

**3.** Verfahren nach Anspruch 1 oder 2, dadurch gekennzeichnet, daß das verwendete Enzym chemisch modifiziert oder ein biosynthetischer Mutant einer natürlichen Form ist.

**4.** Verfahren nach einem jeden der obigen Ansprüche, dadurch gekennzeichnet, daß man ein immobilisiertes Enzym verwendet.

**5.** Verfahren nach einem jeden der obigen Ansprüche, dadurch gekennzeichnet, daß man eine wässrige Reaktionslösung oder -dispersion verwendet, die 0 - 90 %, vorzugsweise 0 - 60 %, eines polaren, wassermischbaren organischen Lösemittels enthält und einen pH-Wert von 3 - 11, vorzugsweise 5 - 10,5, besonders bevorzugt 6 - 10 und insbesondere 7 - 9,5, aufweist.

**6.** Verfahren nach Anspruch 5, dadurch gekennzeichnet, daß das organische Lösemittel aus Alkanolen, Dimethylsulfoxid, Dimethylformamid, Dimethoxyethan und Ethylenglykol ausgewählt ist.

7. Verfahren nach einem jeden der Ansprüche 1 - 4, dadurch gekennzeichnet, daß man eine organische Reaktionslösung oder -dispersion verwendet, die 0 - 10 % Wasser enthält.

8. Verfahren nach Anspruch 7, dadurch gekennzeichnet, daß man ein unpolares organisches Lösemittel verwendet, vorzugsweise ausgewählt aus Dialkylethern, Ethylacetat, Ethylpropionat, Octanen, Heptanen, Hexanen, Petrolethern und Methylenchlorid.

9. Verfahren nach Anspruch 7, dadurch gekennzeichnet, daß man ein flüssiges Substrat oder eine nucleophile Komponente verwendet, die ebenfalls als organisches Lösemittel dienen kann.

10. Verfahren nach einem jeden der obigen Ansprüche, dadurch gekennzeichnet, daß man als Substratkomponente einen D- oder L-Aminosäureester verwendet, der aus Benzylestern oder geradkettigen oder verzweigten $C_1$-$C_8$-Alkylestern, gegebenenfalls mit inerten Substituenten substituiert, ausgewählt ist.

11. Verfahren einem jeden der obigen Ansprüche, dadurch gekennzeichnet, daß man als nucleophile Komponente ein Aminosäureamid der Formel

    $H$-$B$-$NHR^3$

    verwendet, in der $R^3$ Wasserstoff oder $C_1$-$C_3$-Alkyl und B einen L- oder D-Aminocarbonsäurerest bedeuten.

12. Verfahren nach einem jeden der Ansprüche 1 bis 10, dadurch gekennzeichnet, daß man als nucleophile Komponente einen Ester der Formel

    $H$-$B$-$OR^4$

    verwendet, in der B ein L- oder D-Aminocarbonsäurerest und $R^4$ $C_1$-$C_3$-Alkyl bedeutet.

13. Verfahren nach einem jeden der obigen Ansprüche, dadurch gekennzeichnet, daß das erhaltene Dipeptid eine C-terminale Schutzgruppe Y oder mehrere einschließt und daß die Gruppe bzw. Gruppen enzymatisch abgespalten wird/werden, und zwar entweder mittels des gleichen Enzyms, das in der vorherigen Reaktion verwendet wurde, oder mittels eines Enzyms, das eine unterschiedliche Ester- oder Amidspezifität aufweist.

14. Verfahren nach einem jeden der obigen Ansprüche, dadurch gekennzeichnet, daß das erhaltene Dipeptid eine Seitenkettenschutzgruppe oder mehrere einschließt und daß die Gruppe bzw. Gruppen enzymatisch abgespalten wird/werden, vorzugsweise mittels einer Esterase oder Lipase oder eines proteolytischen Enzyms.

**Revendications**

1. Procédé de préparation de dipeptides ou de composés ayant une structure apparentée, ayant la formule générale

    H-A-B-Y

    où A représente un résidu de L- ou D-$\alpha$-amino-acide ou un résidu de $\omega$-amino-acide, éventuellement protégé sur la chaîne latérale, et B représente un résidu d'acide L- ou D-$\alpha$-aminocarboxylique, éventuellement protégé sur la chaîne latérale, qui peut être le même que pour A ou différent de celui-ci, un résidu d'acide L- ou D-aminophosphonique ou un résidu d'acide L- ou D-aminosulfonique ou les $\omega$-amino-acides correspondants ou les sels et hydrates de ceux-ci, et Y représente OH, H, alkyle, aryle, aralkyle ou un groupe bloquant C-terminal, à la condition que A ne puisse pas être Asp ou Gly quand B est Phe et quand Y est un groupe alkyle, ou BY représente un résidu d'amino-alcool

    $B^1$-$CHY^1$-$OH$

où $B^1$ est un dérivé décarboxy des acides aminocarboxyliques tels qu'ils sont définis en relation avec B, et $Y^1$ représente H ou un groupe alkyle, aryle ou aralkyle,
caractérisé en ce qu'on fait réagir un composant de substrat qui est un dérivé d'amino-acide ayant la formule

$H-A-OR^1$ ou 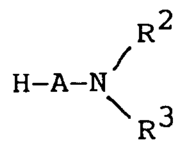

où A est défini comme ci-dessus, $R^1$ représente un groupe alkyle, aryle ou aralkyle éventuellement substitué par des substituants inertes ou un fragment α-désamino d'un amino-acide, et $R^2$ et $R^3$ sont identiques ou différents l'un de l'autre et représentent un atome d'hydrogène ou un groupe alkyle, aryle ou aralkyle éventuellement substitué par des substituants inertes,
avec un composant nucléophile choisi parmi

(a) des amino-acides ayant la formule

H-B-OH

où B est un résidu d'acide aminocarboxylique tel qu'il est défini ci-dessus,
(b) des amides d'amino-acides ayant la formule

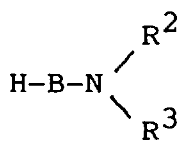

où B est un résidu d'acide aminocarboxylique tel qu'il est défini ci-dessus, et $R^2$ et $R^3$ ont la signification indiquée ci-dessus, sauf que $R^3$ peut aussi représenter un groupe hydroxy ou amino quand $R^2$ représente un atome d'hydrogène,
(c) des esters d'amino-acides ayant la formule

$H-B-OR^4$

où a est un résidu d'acide aminocarboxylique tel qu'il est défini ci-dessus, et $R^4$ représente un groupe alkyle, aryle ou aralkyle,
(d) des acides aminophosphoniques ou des acides aminosulfoniques à chaîne droite ou ramifiée, éventuellement protégés par un groupe d'acide, ayant la formule

$NH_2(CH_2)_xPO_3R^5R_6$ ou $NH_2(CH_2)_xSO_3R^7$

où $R^5$, $R^6$ et $R^7$ représentent, indépendamment les uns des autres, un atome d'hydrogène ou un groupe alkyle, aryle ou aralkyle, et x vaut de 1 à 6,
(e) des aldéhydes ou des cétones d'amino-acides ou leurs dérivés, ayant la formule

$H-B^1-CY^2-R^8$

où $B^1$ est défini comme ci-dessus, $Y^2$ représente O ou un dérivé fonctionnel de celui-ci, de préférence un cétal, et $R^8$ représente H ou un groupe alkyle, aryle ou aralkyle, et
(f) des amino-alcools ayant la formule

$H-B^1-CY^1-OH$

où $B^1$ et $Y^1$ ont la signification indiquée ci-dessus,
en présence d une enzyme amidase ou estérase différente de la sérine ou de la thiolcarboxypeptidase, en solution ou en dispersion, et ensuite, si on le désire, on sépare par clivage un groupe

protecteur présent de la chaîne latérale ou un groupe protecteur Y et/ou, si on le désire, on transforme le dérivé dipeptide résultant en un sel ou un hydrate.

**2.** Procédé selon la revendication 1, caractérisé en ce qu'on utilise une enzyme ayant une activité d'estérase ou d'amidase, qu'on choisit parmi la sérine ou des thiolendoprotéases, des lipases, des estérases et des glycosidases.

**3.** Procédé selon la revendication 1 ou 2, caractérisé en ce que l'enzyme utilisée a été modifiée chimiquement ou est un mutant biosynthétique d'une forme naturelle.

**4.** Procédé selon l'une quelconque des revendications précédentes, caractérisé en ce qu'on utilise une enzyme immobilisée.

**5.** Procédé selon l'une quelconque des revendications précédentes, caractérisé en ce qu'on utilise une solution ou une dispersion aqueuse de réaction contenant de 0 à 90%, de préférence de 0 à 60 %, d'un solvant organique polaire miscible avec l'eau et ayant un pH de 3 à 11, de préférence de 5 à 10,5, et de manière plus préférée de 6 à 10, en particulier de 7 à 9,5.

**6.** Procédé selon la revendication 5, caractérisé en ce qu'on choisit le solvant organique parmi des alcanols, le diméthylsulfoxyde, le diméthylformamide, le diméthoxyéthane et l'éthylèneglycol.

**7.** Procédé selon l'une quelconque des revendications 1 à 4, caractérisé en ce qu'on utilise une solution ou une dispersion organique de réaction contenant de 0 à 10 % d'eau.

**8.** Procédé selon la revendication 7, caractérisé en ce qu'on utilise un solvant organique non polaire, qu'on choisit, de préférence, parmi des éthers dialkyliques, l'acétate d'éthyle, le propionate d'éthyle, des octanes, des heptanes, des hexanes, l'éther de pétrole et le chlorure de méthylène.

**9.** Procédé selon la revendication 7, caractérisé en ce qu'on utilise un substrat liquide ou un composant nucléophile qui peut aussi servir de solvant organique.

**10.** Procédé selon l'une quelconque des revendications précédentes, caractérisé en ce qu'on utilise, comme composant de substrat, un ester de D- ou L-amino-acide, choisi parmi les esters benzyliques ou les esters d'alkyle en $C_1$-$C_8$ à chaîne droite ou ramifiée, éventuellement substitués par des substituants inertes.

**11.** Procédé selon l'une quelconque des revendications précédentes, caractérisé en ce qu'on utilise, comme composant nucléophile, un amide d'amino-acide ayant la formule

$H\text{-}B\text{-}NHR^3$

où $R^3$ est un atome d'hydrogène ou un groupe alkyle en $C_1$-$C_3$,et B est un résidu d'acide L- ou D-aminocarboxylique.

**12.** Procédé selon l'une quelconque des revendications 1 à 10, caractérisé en ce qu'on utilise, comme composant nucléophile, un ester ayant la formule

$H\text{-}B\text{-}OR^4$

où B est un résidu d'acide L- ou D-aminocarboxylique, et $R^4$ est un groupe alkyle en $C_1$-$C_3$.

**13.** Procédé selon l'une quelconque des revendications précédentes, caractérisé en ce que le dipeptide résultant comprend un ou plusieurs groupes protecteurs Y C-terminaux, et que ce groupe ou ces groupes sont séparés enzymatiquement au moyen de la même enzyme que celle utilisée lors de la réaction précédente ou au moyen d'une enzyme ayant une spécificité différente d'ester ou d'amide.

**14.** Procédé selon l'une quelconque des revendications précédentes, caractérisé en ce que le dipeptide résultant comprend un ou plusieurs groupes protecteurs de la chaîne latérale et que ce groupe ou ces

groupes sont séparés enzymatiquement, de préférence au moyen d'une estérase ou d'une lipase ou d'une enzyme protéolytique.